# FASCICULE DE BREVET EUROPEEN

(11) **EP 3 565 884 B1**
(45) Date de publication et mention de la délivrance du brevet: **09.12.2020**
(21) Numéro de dépôt: 18700790.1
(22) Date de dépôt: 05.01.2018
(51) Int. Cl.: C12M 3/06, B01L 3/00, C12M 1/26

(54) **SYSTEME MICROFLUIDIQUE DE MANIPULATION DE CELLULES BIOLOGIQUES**
MIKROFLUIDISCHES SYSTEM ZUR HANDHABUNG VON BIOLOGISCHEN ZELLEN
MICROFLUIDIC SYSTEM FOR HANDLING BIOLOGICAL CELLS

(30) Priorité: 06.01.2017 FR 1750134
(43) Date de publication de la demande: 13.11.2019
(73) Titulaire: Commissariat à l'énergie atomique et aux énergies alternatives, 75015 Paris (FR)
(72) Inventeur: AGACHE, Vincent, 38650 Monestier De Clermont (FR); CASSET, Fabrice, 38570 Tencin (FR); FANGET, Stéphane, 38690 Le Grand Lemps (FR); FOUILLET, Yves, 38340 Voreppe (FR); MILLET, Arnaud, 38340 Voreppe (FR)
(74) Mandataire: Brevalex
(86) Numéro de dépôt international: PCT/FR2018/050022
(87) Numéro de publication internationale: WO 2018/127666

(56) Documents cités:
- US-A1- 2005 014 162
- US-A1- 2006 046 305
- US-A9- 2016 340 644
- ZHANG K ET AL: "A microfluidic system with surface modified piezoelectric sensor for trapping and detection of cancer cells", BIOSENSORS AND BIOELECTRONICS, ELSEVIER BV, NL, vol. 26, no. 2, 15 octobre 2010 (2010-10-15), pages 935-939, XP027320417, ISSN: 0956-5663 [extrait le 2010-07-01]
- A. SHAREI ET AL: "A vector-free microfluidic platform for intracellular delivery", PROCEEDINGS OF THE NATIONAL ACADEMY OF SCIENCES, vol. 110, no. 6, 22 janvier 2013 (2013-01-22), pages 2082-2087, XP055182922, ISSN: 0027-8424, DOI: 10.1073/pnas.1218705110 cité dans la demande

## Description

### DOMAINE TECHNIQUE ET ÉTAT DE LA TECHNIQUE ANTÉRIEURE

La présente invention se rapporte à un système microfluidique de manipulation de cellules biologiques, notamment en vue de leur traitement et/ou de leur analyse.

Dans le domaine de biomédecine, on cherche à pouvoir analyser et traiter les cellules biologiques de manière individuelle. Par exemple, les cellules tumorales circulantes ou CTC sont des cellules tumorales qui quittent la tumeur d'origine et circulent dans l'organisme avant de s'implanter dans un nouvel organe pour former des métastases. La détection et le traitement de ces cellules est un enjeu important. Ces cellules étant peu nombreuses, on souhaite également pouvoir les traiter individuellement et pouvoir évaluer rapidement l'effet du traitement sur celles-ci.

Le document Sangwon Byuna, Sungmin Sonb, Dario Amodeic, Nathan Cermakd, Josephine Shawa, Joon Ho Kange, Vivian C. Hechta, Monte M. Winslowf,g, Tyler Jacksf,h, Parag Mallickc, and Scott R. Manalis. Characterizing deformability and surface friction of cancer cells Proceedings of the National Academy of Sciences 110(19), April 2013*,* décrit un dispositif comportant un résonateur à microcanal suspendu ou SMR (Suspended Microchanel Resonator en terminologie anglo-saxonne) qui permet un comptage et une pesée individuelle des cellules. Le résonateur comporte un microcanal muni d'une portion de section réduite. D'une part la présence d'une cellule dans le microcanal provoque une modification de la fréquence de résonance du résonateur, ce qui permet de détecter la présence d'une cellule, de compter les cellules et d'obtenir certaines informations sur la cellule comme sa masse flottante, sa densité. D'autre part, le temps mis par la cellule pour parcourir la restriction fournit d'autres informations sur la cellule, notamment en lien avec ses propriétés mécaniques, en l'occurrence sa déformabilité. Ces informations permettent d'étudier la nature de la cellule, et d'en déduire leur potentiel métastatique lorsqu'il s'agit d'une cellule tumorale.

Cependant la capacité de ce dispositif est limitée et le dispositif ne permet pas d'agir sur la cellule. Le document US 2006046305 A décrit un système avec une pluralité de canaux, destiné à effectuer un tri de cellules.

Le document A. Sharei et al. "A vector free microfluidic plateform for intracellular delivery", PNAS, February 5, 2013, Vol. 110, n°6*.* décrit un dispositif de délivrance cellulaire ou de transfection comportant plusieurs canaux, comportant chacun une zone de section réduite, pour contraindre les cellules, des pores transitoires peuvent alors être formés, ce qui permet la transfection dans les cellules de macromolécules. Les cellules à traiter et les macromolécules que l'on souhaite transfecter dans les cellules sont introduites à une extrémité des canaux, elles circulent ensemble dans les canaux. Lorsque les cellules pénètrent dans la restriction elles sont contraintes mécaniquement, ce qui fait apparaître des pores par lesquelles les macromolécules peuvent entrer dans les cellules.

Ce dispositif présente pour inconvénient de ne pas permettre une action ciblée sur les cellules. D'une part il n'y a aucun moyen de détecter la présence d'une cellule et donc de décider d'entreprendre ou non une action. D'autre part, il n'est pas possible de réaliser une action sélective de la cellule, en effet toutes les cellules sont transfectées. De plus ce dispositif ne permet que la transfection et aucune autre action.

### EXPOSÉ DE L'INVENTION

C'est par conséquent un but de la présente invention d'offrir un système microfluidique de manipulation de cellules biologiques permettant une action ciblée, individuelle et adaptée sur les cellules, et offrant également une grand modularité dans les actions pouvant être menées sur les cellules.

Le but précédemment énoncé est atteint par un système microfluidique comportant au moins un canal destiné à faire circuler au moins une cellule à manipuler, des moyens de détection de la présence de la cellule, une zone de section réduite inférieure à celle de la cellule et également une zone d'accès à la cellule située dans la zone de section réduite pour permettre d'entreprendre des actions sur la cellule ou vis-à-vis de la cellule.

Les informations collectées par les moyens de détection et éventuellement le temps mis par la cellule à franchir au moins une partie de cette zone à section réduite permettent d'identifier la cellule.

Ainsi les actions entreprises sur les cellules ne sont entreprises que lorsqu'une cellule est détectée et identifiée. Ainsi les actions sont ciblées et utiles car elles n'ont lieu qu'en présence de cellules, en particulier des cellules concernées.

En outre, le fonctionnement du système selon l'invention peut être automatisé au moyen d'une unité de commande générant des ordres d'appliquer une action lorsqu'une cellule est détectée et en fonction de la cellule détectée. Le rendement du système peut donc être amélioré.

La combinaison des moyens de détection, du canal à section réduite et de la zone d'accès permet de disposer d'un système de manipulation optimisé à la fois en fonction et en taille. En effet le canal de section réduite participe à la fois à la détection/ identification de la cellule et à l'action sur la cellule pour la transfection et la collecte de substance.

De manière avantageuse, des deuxièmes moyens de détection en aval de la zone d'accès permettent de caractériser l'effet de l'action appliquée à la cellule dans la zone d'accès, par exemple en comparant les signaux des premiers moyens de détection et les signaux des seconds moyens de détection.

Avantageusement, les premiers et/ou les deuxièmes moyens de détection comportent un support vibrant. Les caractéristiques de l'onde de vibration de la surface sont modifiées par l'arrivée de la cellule et sont dépendantes de la taille de la cellule et de ses propriétés mécaniques. Ces moyens permettent à la fois de détecter la présence d'une cellule mais également de l'identifier au moins partiellement.

La zone de traitement peut par exemple permettre la délivrance de macromolécules aux cellules ou la collecte de substances éjectée par les cellules.

De manière avantageuse, un système d'imagerie est prévu par exemple au niveau de la zone d'accès.

La présente invention a alors pour objet un système de manipulation de cellules biologiques comportant :
- n canaux principaux, n étant un entier au moins égal à 1, chaque canal principal comportant une extrémité d'entrée et une extrémité de sortie, au moins sur une première portion à partir de son entrée d'extrémité, une section transversale telle qu'une cellule circulant dans ladite portion subit des contraintes mécaniques,
- des premiers moyens de détection de la présence d'une cellule au niveau de l'extrémité d'entrée du canal principal,
- au moins une zone d'accès débouchant dans le canal principal entre son extrémité d'entrée et son extrémité de sortie dans ladite première portion, afin de permettre d'exercer une action sur la cellule, ladite zone d'accès étant distincte de l'extrémité d'entrée,
- des moyens de déplacement de la cellule pour contrôler le déplacement la cellule entre l'extrémité d'entrée et l'extrémité de sortie.

Les premiers moyens de détection sont avantageusement aptes à fournir des informations sur au moins une propriété de la cellule.

Les premiers moyens de détection peuvent comporter un support formant une partie d'une paroi du canal principal et au moins un actionneur apte à mettre en vibration ledit support.

Le système de manipulation de cellules biologiques peut également comporte des deuxièmes moyens de détection entre la zone d'accès et l'extrémité de sortie.

Par exemple, la zone d'accès comporte un canal secondaire et des moyens aptes autoriser et à interrompre une communication fluidique entre le canal secondaire et le canal principal. Les moyens aptes autoriser et à interrompre une communication fluidique entre le canal secondaire et le canal principal peuvent comporter un élément d'obturation et sont tels que l'élément d'obturation est soumis dans le canal secondaire à deux pressions s'exerçant en sens opposé, lesdits moyens commandant lesdites pressions pour déplacer l'élément d'obturation.

L'élément d'obturation est de préférence un fluide non miscible avec au moins le liquide contenant la cellule.

Dans un exemple de réalisation, le canal secondaire peut être destiné à être alimenté avec une ou des substances à délivrer à la cellule.

Dans un autre exemple de réalisation, le canal secondaire est destiné à être connecté à une zone de collecte d'une ou de substances éjectées par la cellule. Le canal secondaire peut être destiné à être relié à des moyens d'analyse de la ou des substances éjectées par la cellule.

De manière avantageuse, au moins une partie d'une paroi latérale du canal principal est transparente, le système comportant un système d'imagerie disposé au niveau de ladite partie transparente

De préférence, le système de manipulation comporte une unité de commande reliée au moins aux premiers moyens de détection, à la zone d'accès et aux moyens de contrôle du déplacement de la cellule et aptes à agir au moins sur la zone d'accès et/ou sur les moyens de contrôle du déplacement de la cellule afin d'appliquer une action à la cellule

Avantageusement, le système comporte au moins deux canaux.

L'actionneur peut être un actionneur piézoélectrique.

La présente invention a également pour objet un procédé de manipulation mettant en œuvre un système de manipulation de cellules biologiques selon l'invention comportant :
a) la fourniture d'une solution contenant au moins une cellule à l'extrémité d'entrée du canal principal,
b) déplacement de la cellule dans le canal principal,
c) détection de la présence de la cellule à l'entrée du canal principal,
d) détermination d'au moins une propriété de la cellule et éventuellement détermination de ladite cellule,
e) décision d'appliquer ou non une action à la cellule,
f) si une action est à appliquer à la cellule, commande des moyens de contrôle du déplacement de la cellule pour immobiliser la cellule au niveau de la zone d'accès et commande de la zone d'accès pour appliquer une action,
g) commande des moyens de contrôle du déplacement de la cellule pour l'amener à l'extrémité de sortie.

Dans le cas où le système de manipulation comporte des deuxièmes moyens de détection entre la zone d'accès et l'extrémité de sortie, une comparaison des signaux émis par les premiers moyens de détection et les signaux émis par les deuxièmes moyens de détection peut être réalisée pour détecter une modification d'au moins une propriété de la cellule suite à l'action qui lui a été appliquée.

L'action peut être une délivrance de macromolécules, ou la collecte d'une ou de substances éjectées par la cellule, par exemple le sécrétome.

En considérant la déformabilité de la cellule, l'étape d) peut réaliser une mesure de ladite déformabilité de la cellule et permet de déduire si elle présente un potentiel métastasique.

De préférence au moins les étapes e), f) et g) sont commandées par une unité de commande.

### BRÈVE DESCRIPTION DES DESSINS

La présente invention sera mieux comprise sur la base de la description qui va suivre et des dessins en annexe sur lesquels:
- la figure 1 est une vue en coupe longitudinale schématique d'un exemple de réalisation du système microfluidique de manipulation selon l'invention,
- la figure 2 est une vue en coupe longitudinale schématique d'un exemple de réalisation du système microfluidique de manipulation selon l'invention comportant des moyens de délivrance de macromolécules,
- la figure 3 est une vue schématique de dessus d'un exemple de système microfluidique de manipulation permettant un traitement simultané de plusieurs cellules,
- les figures 4A à 4Q sont des représentations schématiques de différentes étapes d'un exemple d'un procédé de réalisation d'un système microfluidique de manipulation selon l'invention,
- la figure 5 est une représentation en perspective schématique d'un exemple de support vibrant pouvant être mis en œuvre dans le système selon l'invention.

### EXPOSÉ DÉTAILLÉ DE MODES DE RÉALISATION PARTICULIERS

Dans la présente demande, on entend par « cellules biologiques » par exemple des globules blancs ayant un diamètre approximatif compris entre 7 µm et 18µm, des cellules tumorales circulantes ayant un diamètre approximatif d'environ 10µm, des globules rouges ayant un diamètre approximatif compris entre 6 µm et 8µm environ, des bactéries ayant un diamètre approximatif de l'ordre de 2µm. Celles-ci seront désignées par la suite « cellule ».

Les termes « amont » et « aval » sont à considérés dans le sens de circulation principal des cellules dans le système.

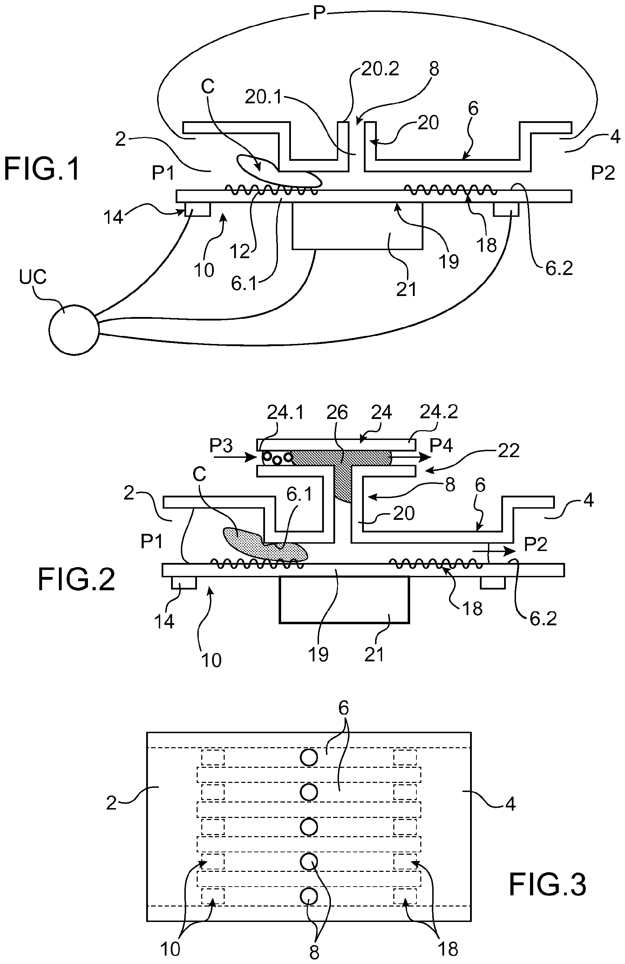

Sur la figure 1, on peut voir une vue en coupe schématique d'un système microfluidique de manipulation de cellules biologiques. Les cellules biologiques sont contenues dans une solution leur permettant de vivre.

Dans la présente demande, le terme « manipulation d'une cellule » signifie toute action sur une cellule visant par exemple à lui délivrer par exemple des macromolécules, à extraire de celles-ci des substances, formant le sécrétome, mais aussi des vésicules Extracellulaires (EVs pour Extracellular vesicles en terminologie anglo-saxonne), parmi lesquels les exosomes, qui auraient pu être sécrétés par la cellule secondairement suite à l'application d'une contrainte mécanique lors du passage dans le canal.

Le système comporte un chemin fluidique qui s'étend, en amont, d'une chambre d'entrée 2 des cellules C destinées à être manipulées à, en aval, une chambre de sortie 4 des cellules biologiques ayant été traitées.

Le système comporte des moyens P de circulation pour faire circuler les cellules entre la chambre d'entrée 2 et la chambre de sortie 4. Pour mettre en mouvement les cellules, une première pression P1 est appliquée dans la chambre d'entrée supérieure à une deuxième pression P2 dans la chambre de sortie. Les pressions P1 et P2 sont commandables comme nous le verrons par la suite. Les moyens de circulation sont par exemple une pompe ou de tout autre moyen bien connu de l'homme du métier, tel qu'un système de pressurisation de réservoirs fluidiques contenant l'échantillon à analyser, et par exemple des tampons biologiques. Les moyens peuvent permettre également de déplacer les cellules de l'aval vers l'amont, par exemple une pompe réversible peut être utilisée, ou en modifiant le différentiel de pression appliqué entre les réservoirs microfluidiques disposés en amont et aval.

La chambre d'entrée 2 est destinée, par exemple, à être connectée à un réservoir (non représenté) contenant la solution contenant les cellules à manipuler et la chambre de sortie 4 est destinée à être connectée à un réservoir (non représenté) de collecte des cellules à traiter.

La chambre d'entrée 4 peut contenir plusieurs cellules C.

Le système comporte au moins un canal principal 6 reliant la chambre d'entrée 2 et la chambre de sortie 4. Le canal principal 6 présente une section transversale inférieure à la section transversale des cellules C à manipuler de sorte que, lorsque les cellules sont forcées à circuler dans le canal principal 6, des contraintes mécaniques leur sont appliquées.

De manière avantageuse, le canal principal 6 présente un diamètre de l'ordre de la moitié de la dimension caractéristique de la cellule en suspension. Par exemple il présente un diamètre compris entre 5 µm et 10 µm en fonction du type cellulaire sur une longueur par exemple comprise entre 10 µm et 40 µm.

Les contraintes appliquées à la cellule ralentissent son déplacement dans le système. En mesurant le temps mis par la cellule pour parcourir une certaine portion du canal principal 6, on peut déterminer de quelle cellule il s'agit. En effet, le temps de transit de la cellule dépend de ses propriétés physiques, liées à sa nature. Des exemples de technique pour mesurer ce temps seront décrits ci-dessous.

Le système comporte une zone d'accès 8 de l'extérieur débouchant dans le canal principal 6.

Le système comporte également des premiers moyens de détection 10 de la présence d'une cellule disposés à l'extrémité d'entrée 6.1 du canal principal 6 lorsqu'une cellule s'est engagée dans le canal principal 6. Les moyens de détection 10 sont avantageusement de type vibrant. Les premiers moyens de détection 10 comportent un support 12 formant une partie de la paroi du canal principal 6 et un ou plusieurs actionneurs 14 aptes à mettre en vibration la surface 12. Les actionneurs sont avantageusement des actionneurs piézoélectriques. En variante, il pourrait s'agir de manière non limitative d'actionneurs électrostatiques, magnétiques, thermiques...

L'activation du ou des actionneurs génèrent une onde de vibration dans le support 12, cette onde est modifiée par la présence d'une cellule, cette modification de l'onde est mesurée et fournit une information sur la présence d'une cellule. Le support vibrant a une fréquence de résonance.

Lorsque la cellule arrive, elle appuie sur le support vibrant, ce qui modifie sa faculté à vibrer. Il en résulte une modification de la fréquence de résonance. Par exemple, en mesurant l'impédance du système, il est possible de déterminer cette modification de fréquence de résonance et donc la modification de l'onde de vibration.

Le support vibrant peut être une plaque apte à générer des ondes de Lamb ou des ondes Rayleigh à l'aide d'actionneurs.

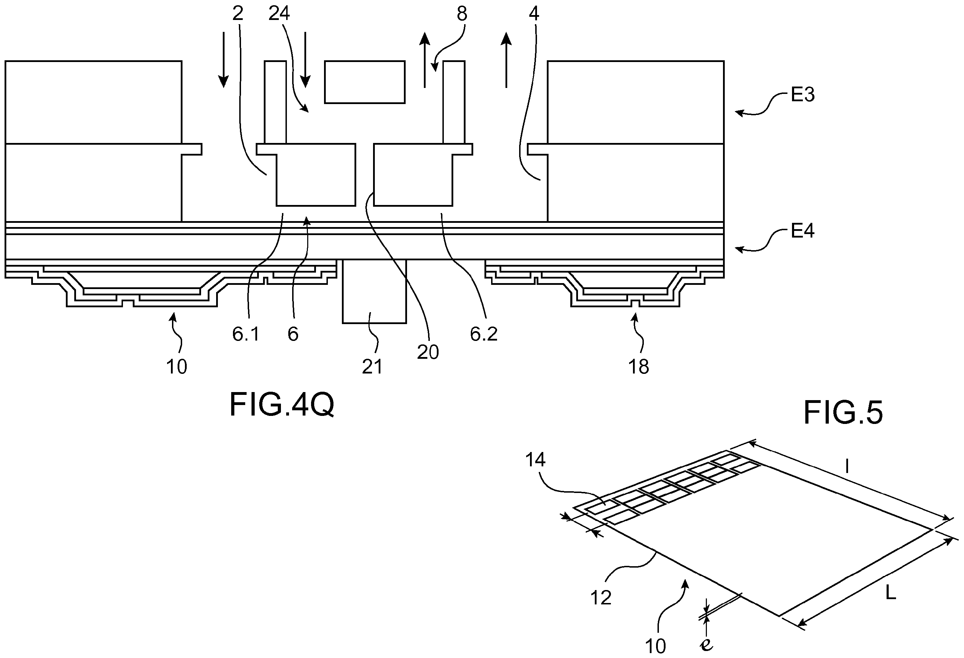

Sur la figure 5, on peut voir un exemple de support vibrant rectangulaire muni d'actionneurs 14 aptes à générer des ondes de Lamb. Par exemple les actionneurs 14 sont répartis en rangée dans la largeur du support et les rangées sont disposées les unes à côté des autres dans la direction de la longueur de la plaque.

Par exemple le support est en verre, dont la largeur L est égale à 61 mm, la longueur l est égale 82 mm et l'épaisseur e est égale 700 µm. Chaque actionneur 14 a une longueur de 9 mm et une largeur de 5 mm et les actionneurs sont séparés les uns des autres de 1 mm dans la direction de la largeur du support et les rangées sont séparées les unes des autres de 4 mm.

De manière avantageuse, le support vibrant 12 est transparent.

De manière avantageuse, des deuxièmes moyens de détection 18 sont prévus en aval de la zone d'accès. Ils sont avantageusement également de type vibrant. Ils permettent de mesurer également une modification de l'onde due à la présence d'une cellule. En comparant les signaux délivrés par les premiers moyens de détection 10 et les deuxièmes moyens de détection 18, il est possible de déterminer si l'action menée dans la zone d'accès 8 a eu un effet sur la cellule. On peut en déduire également si l'action a eu effectivement lieu.

La zone d'accès 8, également appelée « fenêtre » débouche dans la paroi latérale du canal principal 6 en aval des moyens de détection 10. Cette zone d'accès permet d'accéder à la cellule qui circule dans le canal principal 6 et de lui appliquer une action. Il peut s'agir par exemple de transfecter des macromolécules dans la cellule et/ou de prélever une ou des substances émises par la cellule.

Dans l'exemple représenté, la zone d'accès comporte un canal 20 transversal au canal principal 6 et connecté par une extrémité 20.1 au canal principal 6. L'autre extrémité 20.2 du canal transversal 20 est destinée à être connectée à un canal secondaire pouvant être connecté par exemple soit à un réservoir de macromolécules, soit à un réservoir de collecte.

On peut envisager que la section du canal 6 varie, modifiant ainsi les contraintes appliquées à la cellule. Par exemple la section du canal augmente après la zone d'accès.

Il peut également être envisagé plusieurs ensembles en série comportant chacun un canal à section réduite et une zone d'accès pour effectuer plusieurs actions successivement. Par exemple, on peut prévoir un premier ensemble pour récupérer le sécrétome d'une cellule pour l'analyser, puis un deuxième ensemble destiné à transfecter la cellule, par exemple en fonction du sécrétome récupérer dans le premier ensemble et un troisième ensemble pour récupérer le sécrétome après transfection.

De manière également avantageuse, la paroi 19 du canal principal 6 au niveau de la zone d'accès est transparente pour permettre de réaliser des images de la cellule et/ou de l'observer. Un système d'imagerie 21 ou imageur peut alors être disposé à l'extérieur du canal principal 6 en regard de la paroi transparente. Il s'agit par exemple d'une caméra ou d'un imageur CMOS.

Ce système d'imagerie peut permettre de détecter la cellule lorsqu'elle est dans la zone d'accès. Le temps de transit de la cellule peut être mesuré par exemple en suivant le trajet de la cellule par l'image donnée par l'imageur. En déterminant le temps mis par la cellule pour parcourir la distance entre les moyens de détection et le système d'imagerie, on peut identifier la cellule. Une telle identification permet ensuite d'appliquer une action adaptée.

Le temps de parcours de la cellule peut être mesuré également en suivant la fréquence de résonance de la surface vibrante. En effet, le passage de la cellule modifie la fréquence de vibration, ainsi le temps de transit de la cellule dans la restriction de canal peut être déduit du suivi de la fréquence.

De manière avantageuse, l'imageur permet également d'obtenir des images précises de la déformation de la cellule une fois contrainte dans le canal. La déformation, de la cellule est également caractéristique des propriétés et donc de la nature de la cellule. Il est alors possible de corréler le temps de transit de la cellule, i.e. lorsque la cellule franchit la restriction fluidique, mesuré par exemple à partir de la variation de la fréquence de résonance du support vibrant, avec les informations morphologiques obtenues avec l'imageur lorsque la cellule se déforme.

Il pourrait également être envisagé d'utiliser un système d'imagerie supplémentaire en aval de la zone d'accès pour observer par exemple l'effet de l'action sur les cellules.

De manière très avantageuse, le système comporte une unité de commande UC reliée aux premiers moyens de détection, éventuellement aux deuxièmes moyens de détection, et au système d'imagerie. En outre l'unité de commande est apte à commander l'action à appliquer à la cellule. Ainsi l'action peut être déclenchée automatiquement et de manière synchronisée en fonction des signaux émis par les moyens de détection et le système d'imagerie.

Le fonctionnement général du système de manipulation de la figure 1 va maintenant être décrit.

Une ou des cellules sont mise en suspension dans la chambre d'entrée.

La pompe est activée pour amener la cellule jusqu'à l'entrée 6.1 du canal principal et déplacer la cellule dans le canal principal 6.

Au vu de la section du canal principal 6, une seule cellule peut entrer à la fois dans le canal principal 6.

Lorsque la cellule arrivée à l'entrée du canal principal 6, elle entre en contact avec le support vibrant des premiers moyens de détection 10. Le support vibrant présente un mode de vibration, comme par exemple une onde de surface, telle que les ondes de Rayleigh ou de Lamb.

L'onde de surface est modifiée par la présence de la cellule. En effet la cellule induit une variation de raideur ou de masse du support vibrant la fréquence est alors décalée car la fréquence est proportionnelle à V(k/m).

La présence d'une cellule à l'entrée du canal principal est donc détectée par cette modification de l'onde de surface.

En outre, à partir des variations des propriétés de la surface vibrante, les propriétés mécaniques de la cellule pourront être déduites, telles que la masse, l'élasticité. La densité peut également être déduite de ces variations en connaissant la densité de la solution.

De manière très avantageuse, du fait de la section réduite du canal principal 6, la cellule se déforme en se déplaçant dans celui-ci, ce qui permet d'optimiser la mesure de la déformabilité de la cellule via l'image de la cellule déformée donnée par l'imageur. Cette information permet de déduire le potentiel métastatique dans le cas des Cellules Tumorales Circulantes.

Le support vibrant offre en outre une assistance au déplacement de la cellule dans le canal principal 6.

La présence d'une cellule est détectée et cette cellule est identifiée ou au moins une partie de ses propriétés est déterminée.

L'unité de traitement peut alors générer des ordres pour appliquer une action sur la cellule au niveau de la zone d'accès. Cette action peut être, par exemple, soit une transfection, soit une collecte de substance éjectée par la cellule.

D'une part le système est plus efficace car il agit lorsqu'une cellule est présente et il est capable d'appliquer une action adaptée à la cellule en fonction de ses propriétés.

Lorsque la cellule a subi ou non une action au niveau de la zone d'accès, elle poursuit son déplacement en direction de la chambre de sortie. Elle entre alors en contact avec les deuxièmes moyens de détection. Les modifications de l'onde de surface permettent également d'obtenir des informations sur les propriétés de la cellule. En comparant ces propriétés à celles déterminées avec les informations fournies par les premiers moyens de détection, il est possible de déterminer si l'action a eu lieu et/ou a eu un effet sur la cellule.

Par exemple s'il est détecté que l'action n'a pas eu effet, il peut être décidé de renvoyer la cellule vers la zone d'accès pour lui faire à nouveau subir l'action, en inversant le sens de déplacement imposé par le système de pompage ou de pressurisation microfluidique. En variante, la cellule peut être acheminée vers une seconde zone d'accès en aval de la zone d'accès 8 pour lui appliquer une nouvelle action On peut envisager en variante de renvoyer la cellule vers la zone d'accès pour lui appliquer une autre action.

La cellule se déplace jusqu'à la chambre de sortie pour être collectée ou évacuée.

Grâce à l'invention, l'ensemble du processus subi par la cellule peut être automatisé.

Sur la figure 2, on peut voir un exemple de système adapté à la transfection.

Dans cet exemple, le canal transversal 20 de la zone d'accès comporte au niveau de son extrémité 20.2 des moyens de connexion au réservoir de macromolécules.

Les moyens de connexion 22 comportent un canal secondaire 24 munie d'une première extrémité 24.1 et d'une deuxième extrémité 24.2. L'extrémité 20.2 du canal transversal 20 débouche dans la paroi latérale du canal secondaire 24 entre sa première extrémité 24.1 et sa deuxième extrémité 24.2. La première extrémité 24.1 est destinée à être connecté à un réservoir de macromolécules ou toute autre substance à transfecter.

La première extrémité 24.1 est soumise à une troisième pression P3 et la deuxième extrémité 24.2 est soumise à une quatrième pression P4.

En outre un moyen d'obturation 26 est disposé entre les extrémités 20.2, 24.1 et 24.2 de sorte à isoler les extrémités 20.1, 24.1 et 24.2 les unes des autres. Le moyen d'obturation est par exemple formé par de l'huile ou tout autre matériau liquide non miscible avec les solutions contenant les cellules et les macromolécules.

En variante, tout autre moyen de connexion, du type vanne hydraulique, pourrait être mis en œuvre.

Le déroulement de la transfection est le suivant :
Initialement les pressions P3, P4 et la pression dans le canal transversal 20 sont telles que le moyen d'obturation 26 isole la solution contenant les macromolécules du canal transversal 20 et du canal principal 6.

D'une part, grâce au signal transmis par les moyens de détection 10, l'unité de commande est informée de la présence d'une cellule à l'entrée du canal principal 6. L'unité de commande peut alors déclencher la transfection. Le système selon l'invention permet donc une synchronisation entre la détection et le déclenchement de l'action.

Pour cela la cellule C est immobilisée au niveau de la zone d'accès de sorte à être en regard de l'extrémité 20.1 du canal transversal 20. Par exemple les pressions P1 et P2 sont rendues égales ou la pression P1 est abaissée suffisamment pour que la cellule s'immobilise et ferme la première extrémité 20.1 du canal transversal 20.

Ensuite, les pressions P3 et P4 sont commandées pour que le moyen d'obturation 26 se déplace vers la deuxième extrémité 24.2 et mette en communication la première extrémité 24.1 et le canal transversal 20. En outre, la pression P3 est commandée de sorte à être supérieure à celle dans le canal transversal 20 provoquant un écoulement de la solution contenant les macromolécules vers le canal transversal 20. Les macromolécules viennent en contact avec la portion de la membrane de la cellule fermant l'extrémité 20.1. En outre du fait des contraintes mécaniques appliquées à la cellule par la section réduite du canal principal 6, des pores avantageusement provisoires sont formés dans la membrane permettant la délivrance de macromolécules dans la cellule.

Lorsque la délivrance est terminée, les pressions P3 et P4 sont modifiées de sorte à remettre l'élément d'obturation 26 en position initiale isolant le tube transversal 20 de la première extrémité 24.1 et la cellule est de nouveau déplacée en direction de la chambre de sortie 4. Les pores, lorsqu'ils sont provisoires, se referment lorsque la cellule sort du canal principal 6.

Il peut être prévu de soumettre la cellule à divers stimuli comme des cytokines, des molécules facilitant la sécrétion d'exosomes comme le calcium ionophore.

Une autre action qui peut être menée au niveau de la zone d'accès est la collecte de substances émises par la cellule soumise aux contraintes mécaniques appliquées par le canal à section réduite 6.

La cellule éjecte des substances qui se retrouvent dans le canal secondaire 20. En commandant les pressions P1, P2, P3 et P4 les substances peuvent être collectées au niveau de la première extrémité 24.1 ou la deuxième extrémité 24.2 du canal secondaire 24.

La ou les substances éjectées par la cellule, du fait de la contrainte qui lui a été appliquée en la forçant à passer dans le canal principal 6, peut ou peuvent être du sécrétome, comportant notamment les vésicules Extracellulaires (EVs) (parmi lesquels les exosomes décrites dans Sheridan C. Exosome cancer diagnostic reaches market Nat Biotechnol. 2016 Apr;34(4):359-60*.* ]

Ces substances peuvent être analysées et/ou collectés.

Dans un exemple avantageux, on peut associer à la zone d'accès des moyens d'analyse des substances éjectées par la cellule et récupérées. Par exemple, les moyens d'analyse peuvent être un spectromètre de masse (MALDI-TOF-MS/MS :Matrix-assisted laser desorption/ionization-Time of Flight-Mass spectrometry en terminologie anglo-saxonne), un marqueur fluorescent, un dispositif d'analyse par réaction en chaîne par polymérase ou PCR (Polymerase Chain Reaction en terminologie anglo-saxonne), protéomique, un dispositif de comptage et pesée d'EVs par résonateur suspendu à nanocanal ou SNR (Suspended Nanochannel Resonator en terminologie anglo-saxonne), un dispositif microfluidique passif du type à déplacement latéral déterministe ou DLD (Deterministic Lateral Displacement en terminologie anglo-saxonne) pour isoler par gamme de taille des sous-populations de vésicules extracellulaires (dont la taille varie entre 30nm à 1µm), RAMAN (parmi ces méthodes celles à base de spectroscopie de Raman, amplifiée par la surface ou SERS (Surface Enhanced Raman Spectroscopy en terminologie anglo-saxonne), cytométrie en flux, diffusion dynamique de la lumière ou DLS (Diffusion Light Scattering en terminologie anglo-saxonne).

Dans le cas de cellules adhérentes, celles-ci sécrètent des protéines de la matrice extracellulaire, dont l'étude structurale et fonctionnelle est très importante dans l'analyse des cellules.

Par ailleurs, du fait de la section réduite du canal principal 6, la cellule est contrainte à l'entrée du canal. La déformabilité des cellules associées à la réduction de la section du canal peut avantageusement être un élément de caractérisation de l'état de la cellule. On peut avantageusement analyser cette déformabilité, par exemple par microscopie optique, par exemple pour mesurer le temps de passage dans un canal constrictif.

Grâce au système selon l'invention, on peut envisager d'étudier l'effet d'une contrainte mécanique appliquée à la cellule par le canal principal 6, sur la modification de la sécrétion des exosomes d'une cellule stimulée ou non chimiquement, en mettant en œuvre des moyens de collecte et des moyens d'analyse de ces exosomes dans la zone d'accès comme cela a été décrit ci-dessus.

Sur la figure 3, on peut voir une vue de dessus schématique d'un système microfluidique selon l'invention comportant plusieurs canaux 6 parallèles connectés à la chambre d'entrée et à la chambre de sortie. Les canaux sont alimentés en parallèles et il est possible d'appliquer une même action ou des actions différentes à plusieurs cellules simultanément.

En variante, chaque canal pourrait avoir sa propre chambre d'entrée séparée des autres chambres d'entrée et/ou sa propre chambre de sortie séparée des autres chambres de sortie.

Les canaux peuvent avoir la même section et servir par exemple à manipuler les mêmes cellules ou même type de cellule, ou ils peuvent avoir des sections différentes et manipuler des cellules de tailles différentes.

Tout ou partie des canaux peuvent partager la même zone d'accès, la même action étant alors appliquée à tout ou partie des cellules. Ou chaque canal a sa propre zone d'accès, ce qui permet une action différente pour chaque canal.

La mise œuvre de plusieurs canaux permet d'avoir une redondance des informations obtenues sur les cellules, d'augmenter le débit d'analyse, le débit de traitement, le débit de collecte....

A tire d'exemple, le système pourrait comporter 10 canaux en parallèle, chaque canal étant piloté individuellement par un moyen de régulation microfluidique, tel qu'une pompe ou un système de pressurisation de réservoirs.

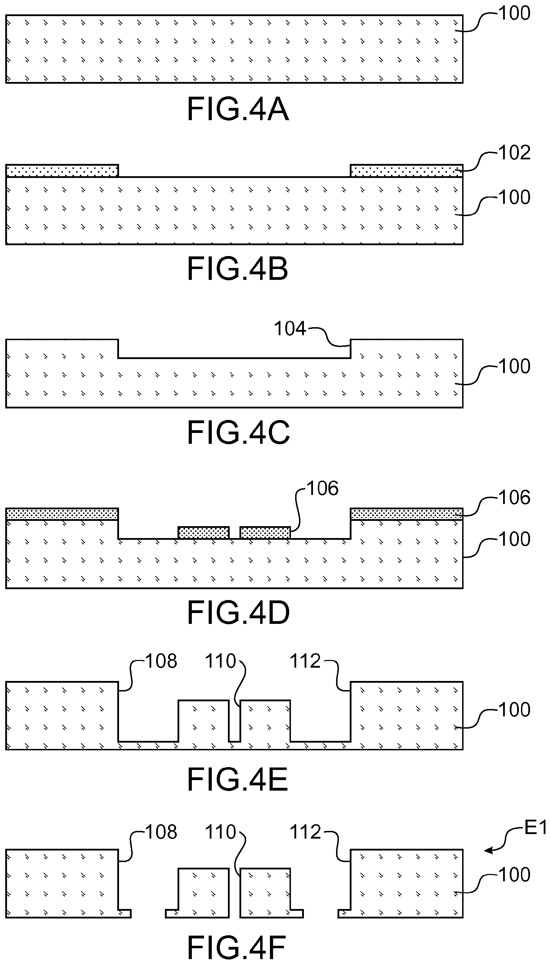

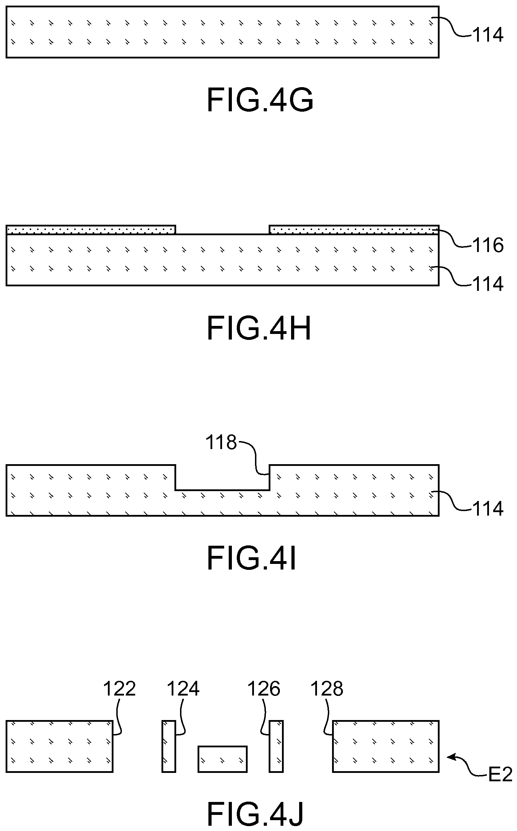

Un exemple de procédé de réalisation du système de manipulation de cellules selon l'invention va maintenant être décrit à l'aide des figures 4A à 4Q.

Le dispositif sera par exemple réalisé en verre, mais d'autres matériaux peuvent être utilisés par exemple du PDMS.

Un substrat en verre 100 est représenté sur la figure 4A
Lors d'une première étape, on forme un masque par lithographie 102 en face avant du substrat 100 (figure 4B), de sorte à créer une première cavité 104 en face avant par gravure. Le masque est ensuite retiré.

L'élément ainsi obtenu est représenté sur la figure 4C.

Lors d'une étape suivante on dépose un autre masque 106 afin de structurer la cavité 104.

L'élément ainsi obtenu est représenté sur la figure 4D.

Lors d'une étape suivante, le fond de la cavité est structuré de sorte à former trois autres cavités 108, 110, 112, par exemple par gravure.

L'élément ainsi obtenu est représenté sur la figure 4E.

Lors d'une étape suivante, on structure le fond des cavités 108, 110 et 112, par exemple par gravure, de sorte à ce qu'elles débouchent en face arrière du substrat 100.

L'élément E1 ainsi obtenu est représenté sur la figure 4F.

On utilise ensuite un autre substrat 114, par exemple également en verre (figure 4G).

Lors d'une étape suivante, on forme un masque 116 en face avant du substrat 114 (figure 4H), de sorte à créer une première cavité 118 en face avant par gravure. Le masque est ensuite retiré.

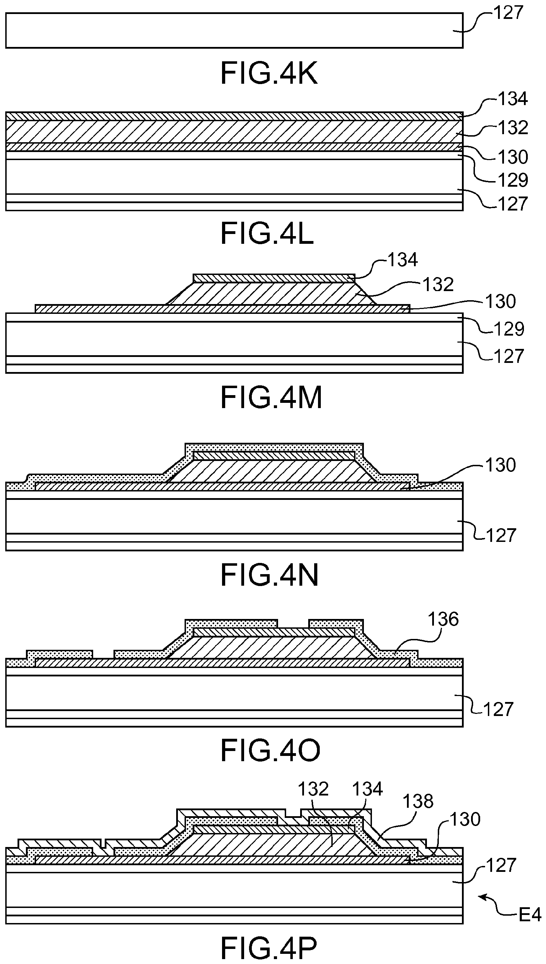

L'élément ainsi obtenu est représenté sur la figure 4l.

Lors d'une étape suivante, on forme un autre masque 120 sur la face avant du substrat 114 et sur une partie du fond de la cavité 118.

Lors d'une étape suivante, le substrat est structuré de sorte à former quatre cavités débouchantes 122, 124, 126, 128 dans la face arrière du substrat 114. Les cavités 122 et 128 sont situées de part et d'autre de la 118 et les cavités 124, 126 sont situées dans la cavité 118.

L'élément E2 ainsi obtenu est représenté sur la figure 4J.

Lors d'une étape suivante, on assemble les éléments E1 et E2, la face avant de l'élément E2 étant en contact avec la face arrière de l'élément E1 et de sorte que les cavités 108 et 112 débouchent dans les cavités 122 et 128 et la cavité 110 débouche dans la cavité 118. L'assemblage est par exemple réalisé par scellement anodique ou scellement moléculaire. On obtient un élément E3 visible sur la figure 4Q.

On réalise par ailleurs un support destiné à fermer le canal 6 et comportant les premiers moyens de détection 10, et les deuxièmes moyens de détection et une zone transparente pour un système d'imagerie.

Dans la description qui suit, seule la réalisation des premiers moyens de détection est décrite, mais la réalisation des deuxièmes moyens de détection peut être simultanée à celle des premiers moyens, et les deuxièmes moyens de détection ont une structure proche de celle des premiers moyens.

On utilise un autre substrat 127, par exemple en verre (figure 4K).

On réalise les actionneurs piézoélectriques par :
- formation d'une couche 130 destinée à former l'une des électrodes, par exemple en Mo sur la face avant du substrat 127, par exemple par dépôt pleine plaque,
- formation d'une couche 132 de matériau piézoélectrique par exemple du PZT, de l'AIN, du ZnO,... par exemple par dépôt pleine plaque, et
- formation d'une autre couche 134 destinée à former l'autre électrode, par exemple en MO, par exemple par dépôt pleine plaque.

Avantageusement une couche 129 destiné à orienter le matériau de le la couche d'électrode 130 en vue d'orienter le matériau piézoélectrique est formée préalablement au dépôt de la couche 130. Par exemple la couche 129 est une couche fine d'AIN sur laquelle est déposé le Mo, puis l'AIN.

L'élément ainsi obtenu est représenté sur la figure 4L.

Lors d'une étape suivante, les couches 130, 132 et 134 sont structurées successivement, par exemple par gravure.

L'élément ainsi obtenu est représenté sur la figure 4M.

Lors d'une étape suivante, une couche de passivation 136 est formée sur la face avant de l'élément de la figure 4N, par exemple une couche d'oxyde, par exemple d'épaisseur 300 nm. Par exemple la couche de passivation est de l'oxyde SiO₂ déposé par la technique de dépôt chimique en phase vapeur assisté par plasma ou PECVD (plasma enhanced chemical vapor déposition en terminologie anglo-saxonne).

L'élément ainsi obtenu est représenté sur la figure 4N.

Lors d'une étape suivante, la couche 136 est structurée, par exemple par photolithographie et gravure, pour ouvrir la couche 136 au niveau des électrodes et permettre la réalisation de contact.

L'élément ainsi obtenu est représenté sur la figure 4O.

Lors d'une étape suivante, un couche conductrice électrique 138, par exemple en or, est formée sur la couche d'oxyde et vient en contact avec les électrodes au niveau des ouvertures dans la couche 136.

L'élément E4 ainsi obtenu est représenté sur la figure 4P.

L'élément E4 est ensuite assemblé avec l'élément E3 de sorte que les faces arrière des deux éléments soient en contact. Les éléments E3 et E4 sont assemblés par exemple en déposant une colle procédé de sérigraphie de manière à être localisée uniquement au niveau des zones de collage ou alors par un collage anodique des deux faces de verre des éléments E3 et E4.

On obtient alors le système de manipulation selon l'invention (figure 4Q).

Dans cet exemple les actionneurs sont à l'extérieur du canal mais il pourrait être prévu qu'ils soient à l'intérieur du canal

Dans l'exemple représenté la zone entre les deux actionneurs permet la mise en place d'un dispositif d'imagerie, par exemple de type CMOS.

## Revendications

1. Système de manipulation de cellules biologiques comportant :
- n canaux principaux (6), n étant un entier au moins égal à 1, avantageusement n étant un entier au moins égal à deux, chaque canal principal (6) comportant une extrémité d'entrée (6.1) et une extrémité de sortie (6.2), au moins sur une première portion à partir de son entrée d'extrémité (6.1), une section transversale telle qu'une cellule (C) circulant dans ladite portion subit des contraintes mécaniques,
- des premiers moyens de détection (10) de la présence de ladite cellule au niveau de l'extrémité d'entrée du canal principal (6),
- au moins une zone d'accès (8) débouchant dans le canal principal (6) entre son extrémité d'entrée (6.1) et son extrémité de sortie (6.2) dans ladite première portion, afin de permettre d'exercer une action sur ladite cellule (C), ladite zone d'accès (8) étant distincte de l'extrémité d'entrée (6.1),
- des moyens de déplacement (P) de ladite cellule pour contrôler le déplacement la cellule entre l'extrémité d'entrée (6.1) et l'extrémité de sortie (6.2), et de sorte que la cellule entre dans le canal principal par l'extrémité d'entrée (6.1) et sorte du canal principal par l'extrémité de sortie (6.2).

2. Système de manipulation de cellules biologiques selon la revendication 1, dans lequel les premiers moyens de détection (10) sont aptes à fournir des informations sur au moins une propriété de la cellule.

3. Système de manipulation de cellules biologiques selon la revendication 1 ou 2, dans lequel les premiers moyens de détection (10) comportent un support formant une partie d'une paroi du canal principal (6) et au moins un actionneur (14) apte à mettre en vibration ledit support.

4. Système de manipulation de cellules biologiques selon la revendication 1, 2 ou 3, comportant des deuxièmes moyens de détection entre la zone d'accès (8) et l'extrémité de sortie (6.2).

5. Système de manipulation de cellules biologiques selon l'une des revendications 1 à 4, dans lequel la zone d'accès (8) comporte un canal secondaire (24) et des moyens aptes autoriser et à interrompre une communication fluidique entre le canal secondaire (24) et le canal principal (6).

6. Système de manipulation de cellules biologiques selon la revendication 5, dans lequel les moyens aptes autoriser et à interrompre une communication fluidique entre le canal secondaire (24) et le canal principal (6) comportent un élément d'obturation (26) et sont tels que l'élément d'obturation (26) est soumis dans le canal secondaire (24) à deux pressions s'exerçant en sens opposé, lesdits moyens commandant lesdites pressions pour déplacer l'élément d'obturation (26), l'élément d'obturation (26) étant avantageusement un fluide non miscible avec au moins le liquide contenant la cellule.

7. Système de manipulation de cellules biologiques selon la revendication 5 ou 6, dans lequel le canal secondaire (24) est destiné à être alimenté avec une ou des substances à délivrer à la cellule, ou à être connecté à une zone de collecte d'une ou de substances éjectées par la cellule.

8. Système de manipulation de cellules biologiques selon la revendication 7, dans lequel le canal secondaire(24) est destiné à être relié à des moyens d'analyse de la ou des substances éjectées par la cellule.

9. Système de manipulation selon l'une des revendications précédentes, dans lequel au moins une partie d'une paroi latérale du canal principal (6) est transparente, le système comportant un système d'imagerie disposé au niveau de ladite partie transparente.

10. Système de manipulation selon l'une des revendications 1 à 9, comportant une unité de commande (UC) reliée au moins aux premiers moyens de détection (10), à la zone d'accès (8) et aux moyens de contrôle du déplacement (P) de la cellule et aptes à agir au moins sur la zone d'accès (8) et/ou sur les moyens de contrôle du déplacement (P) de la cellule afin d'appliquer une action à la cellule

11. Système de manipulation selon l'une des revendications 1 à 10 en combinaison avec la revendication 3, dans lequel ledit au moins un actionneur (14) est un actionneur piézoélectrique.

12. Procédé de manipulation mettant en œuvre un système de manipulation de cellules biologiques selon l'une des revendications 1 à 11 comportant :
a) la fourniture d'une solution contenant au moins une cellule à l'extrémité d'entrée du canal principal,
b) le déplacement de la cellule dans le canal principal,
c) la détection de la présence de la cellule à l'entrée du canal principal,
d) la détermination d'au moins une propriété de la cellule et éventuellement détermination de ladite cellule,
e) la décision d'appliquer ou non une action à la cellule,
f) si une action est à appliquer à la cellule, la commande des moyens de contrôle du déplacement de la cellule pour immobiliser la cellule au niveau de la zone d'accès et commande de la zone d'accès pour appliquer une action,
g) la commande des moyens de contrôle du déplacement de la cellule pour l'amener à l'extrémité de sortie,
avantageusement au moins les étapes e), f) et g) étant commandées par une unité de commande.

13. Procédé de manipulation selon la revendication 12, le système de manipulation comportant des deuxièmes moyens de détection entre la zone d'accès et l'extrémité de sortie, dans lequel une comparaison des signaux émis par les premiers moyens de détection et les signaux émis par les deuxièmes moyens de détection est réalisé pour détecter une modification d'au moins une propriété de la cellule suite à l'action qui lui a été appliquée.

14. Procédé de manipulation selon la revendication 12 ou 13, dans lequel l'action est une délivrance de macromolécules ou l'action est la collecte d'une ou de substances éjectées par la cellule, par exemple le sécrétome.

15. Procédé de manipulation selon l'une des revendications 12 à 14, ladite au moins une propriété de la cellule étant sa déformabilité, l'étape d), réalise une mesure de ladite déformabilité de la cellule et permet de déduire si elle présente un potentiel métastasique.

## Patentansprüche

1. System zur Handhabung von biologischen Zellen, umfassend:
- n Hauptkanäle (6), wobei n eine ganze Zahl wenigstens gleich 1 ist, wobei n vorzugsweise eine ganze Zahl wenigstens gleich 2 ist, wobei jeder Hauptkanal (6) ein Eingangsende (6.1) und ein Ausgangsende (6.2) umfasst, sowie auf wenigstens einem ersten Bereich ausgehend von seinem Eingangsende (6.1) einen derartigen Querschnitt, dass eine Zelle (C), die in dem Bereich zirkuliert, mechanischen Beanspruchungen ausgesetzt ist,
- erste Mittel (10) zur Detektion des Vorhandenseins der Zelle im Bereich des Eingangsendes des Hauptkanals (6),
- wenigstens eine Zugangszone (8), die in den Hauptkanal (6) zwischen seinem Eingangsende (6.1) und seinem Ausgangsende (6.2) in dem ersten Bereich mündet, um zu ermöglichen, dass eine Aktion an der Zelle (C) ausgeübt wird, wobei die Zugangszone (8) von dem Eingangsende (6.1) verschieden ist,
- Mittel (P) zur Verlagerung der Zelle zum Steuern der Verlagerung der Zelle zwischen dem Eingangsende (6.1) und dem Ausgangsende (6.2) und derart, dass die Zelle am Eingangsende (6.1) in den Hauptkanal eintritt und am Ausgangsende (6.2) aus dem Hauptkanal austritt.

2. System zur Handhabung von biologischen Zellen nach Anspruch 1, bei dem die ersten Detektionsmittel (10) dazu ausgelegt sind, Informationen über wenigstens eine Eigenschaft der Zelle zu liefern.

3. System zur Handhabung von biologischen Zellen nach Anspruch 1 oder 2, bei dem die ersten Detektionsmittel (10) einen Träger umfassen, der einen Teil einer Wand des Hauptkanals (6) bildet, und wenigstens ein Betätigungselement (14), das dazu ausgelegt ist, den Träger in Vibration zu versetzen.

4. System zur Handhabung von biologischen Zellen nach Anspruch 1, 2 oder 3, umfassend zweite Detektionsmittel zwischen der Zugangszone (8) und dem Ausgangsende (6.2).

5. System zur Handhabung von biologischen Zellen nach einem der Ansprüche 1 bis 4, bei dem die Zugangszone (8) einen Sekundärkanal (24) und Mittel umfasst, die dazu ausgelegt sind, eine fluidische Kommunikation zwischen dem Sekundärkanal (24) und dem Hauptkanal (6) zuzulassen und zu unterbrechen.

6. System zur Handhabung von biologischen Zellen nach Anspruch 5, bei dem die Mittel, die dazu ausgelegt sind, eine fluidische Kommunikation zwischen dem Sekundärkanal (24) und dem Hauptkanal (6) zuzulassen und zu unterbrechen, ein Verschlusselement (26) umfassen und derart sind, dass das Verschlusselement (26) in dem Sekundärkanal (24) zwei Drücken ausgesetzt ist, die in entgegengesetzter Richtung wirken, wobei die Mittel die Drücke steuern, um das Verschlusselement (26) zu verlagern, wobei das Verschlusselement (26) vorzugsweise ein Fluid ist, das wenigstens mit der Flüssigkeit, die die Zelle enthält, nicht mischbar ist.

7. System zur Handhabung von biologischen Zellen nach Anspruch 5 oder 6, bei dem der Sekundärkanal (24) dazu ausgelegt ist, mit einer oder mehreren zu der Zelle zu liefernden Substanzen versorgt zu werden, oder mit einer Zone zum Sammeln von einer oder mehreren durch die Zelle ausgestoßenen Substanzen verbunden zu werden.

8. System zur Handhabung von biologischen Zellen nach Anspruch 7, bei dem der Sekundärkanal (24) dazu ausgelegt ist, mit Mitteln zur Analyse der durch die Zelle ausgestoßenen Substanz(en) verbunden zu sein.

9. System zur Handhabung nach einem der vorhergehenden Ansprüche, bei dem wenigstens ein Teil einer lateralen Wand des Hauptkanals (6) transparent ist, wobei das System ein Bildgebungssystem umfasst, das im Bereich des transparenten Teils angeordnet ist.

10. System zur Handhabung nach einem der Ansprüche 1 bis 9, umfassend eine Steuereinheit (UC), die wenigstens mit den ersten Detektionsmitteln (10), mit der Zugangszone (8) und mit den Mitteln (P) zur Steuerung der Verlagerung der Zelle verbunden und dazu ausgelegt ist, wenigstens auf die Zugangszone (8) und/oder auf die Mittel (P) zur Steuerung der Verlagerung der Zelle einzuwirken, um eine Aktion auf die Zelle auszuüben.

11. System zur Handhabung nach einem der Ansprüche 1 bis 10 in Kombination mit Anspruch 3, bei dem das wenigstens eine Betätigungselement (14) ein piezoelektrisches Betätigungselement ist.

12. Verfahren zur Handhabung, das ein System zur Handhabung von biologischen Zellen nach einem der Ansprüche 1 bis 11 verwendet, umfassend:
a) Bereitstellen einer Lösung, die wenigstens eine Zelle enthält, am Eingangsende des Hauptkanals,
b) Verlagern der Zelle in dem Hauptkanal,
c) Detektion des Vorhandenseins der Zelle am Eingang des Hauptkanals,
d) Bestimmung wenigstens einer Eigenschaft der Zelle und gegebenenfalls Bestimmung der Zelle,
e) Entscheiden, ob oder ob nicht eine Aktion an der Zelle ausgeführt wird,
f) wenn eine Aktion an der Zelle ausgeführt werden soll, Steuern der Mittel zur Steuerung der Verlagerung der Zelle, um die Zelle im Bereich der Zugangszone zu immobilisieren, und Steuern der Zugangszone, um eine Aktion auszuführen,
g) Steuern der Mittel zur Steuerung der Verlagerung der Zelle, um sie zum Ausgangsende zu bringen,
wobei vorzugsweise wenigstens die Schritt e), f) und g) durch eine Steuereinheit gesteuert werden.

13. Verfahren zur Handhabung nach Anspruch 12, wobei das System zur Handhabung zweite Detektionsmittel zwischen der Zugangszone und dem Ausgangsende umfasst, wobei ein Vergleich der Signale, die von den ersten Detektionsmitteln emittiert werden, mit den Signalen durchgeführt wird, die von den zweiten Detektionsmitteln emittiert werden, um eine Veränderung wenigstens einer Eigenschaft der Zelle im Anschluss an die Aktion zu detektieren, die an ihr ausgeführt wurde.

14. Verfahren zur Handhabung nach Anspruch 12 oder 13, bei dem die Aktion eine Zuführung von Makromolekülen ist, oder die Aktion das Sammeln von einer oder mehreren Substanzen ist, die durch die Zelle ausgestoßen werden, beispielsweise Sekretom.

15. Verfahren zur Handhabung nach einem der Ansprüche 12 bis 14, wobei die wenigstens eine Eigenschaft der Zelle ihre Verformbarkeit ist, wobei der Schritt d) eine Messung der Verformbarkeit der Zelle durchführt und eine Schlussfolgerung erlaubt, ob sie ein Metastasenpotential aufweist.

## Claims

1. System for handling biological cells comprising:
- n main channels (6), n being a whole number at least equal to 1, advantageously n being a hole number at least equal tot 2, with each main channel (6) comprising an inlet end (6.1) and an outlet end (6.2), at least on a first portion starting from its end inlet (6.1), a transverse section such as a cell (C) circulating in said portion undergoes mechanical stresses,
- first means for detecting (10) the presence of said cell on the inlet end of the main channel (6),
- at least one access zone (8) opening into the main channel (6) between its inlet end (6.1) and its outlet end (6.2) in said first portion, in order to make it possible to exert an action on said cell (C), said access zone (8) being distinct from the inlet end (6.1),
- means for displacing (P) said cell for controlling the displacement of the cell between the inlet end (6.1) and the outlet end (6.2), and in such a way that the cell enters into the main channel by the inlet end (6.1) and exits from the main channel by the outlet end (6.2).

2. System for handling biological cells according to claim 1, wherein the first means for detecting (10) are able to provide information on at least one property of the cell.

3. System for handling biological cells according to claim 1 or 2, wherein the first means for detecting (10) comprise a support forming a portion of the wall of the main channel (6) and at least one actuator (14) able to put into vibration said support.

4. System for handling biological cells according to claim 1, 2 or 3, comprising second means for detecting between the access zone (8) and the outlet end (6.2).

5. System for handling biological cells according to one of claims 1 to 4, wherein the access zone (8) comprises a secondary channel (24) and means able to authorise and to interrupt a fluidic communication between the secondary channel (24) and the main channel (6).

6. System for handling biological cells according to claim 5, wherein the means able to authorise and to interrupt a fluidic communication between the secondary channel (24) and the main channel (6) comprise a closing element (26) and are such that the closing element (26) is subjected in the secondary channel (24) to two pressures being exerted in the opposite direction, said means controlling said pressures in order to displace the closing element (26), the closing element (26) being advantageously a fluid that is immiscible with at least the liquid that contains the cell.

7. System for handling biological cells according to claim 5 or 6, wherein the secondary channel (24) is intended to be supplied with one or several substances to be delivered to the cell or to be connected to a zone for collecting one or several substances ejected by the cell.

8. System for handling biological cells according to claim 7, wherein the secondary channel (24) is intended to be connected to means for analysing the substance or substances ejected by the cell.

9. System for handling according to one of the preceding claims, wherein at least one portion of a side wall of the main channel (6) is transparent, with the system comprising an imaging system arranged on said transparent portion.

10. System for handling according to one of claims 1 to 9, comprising a control unit (UC) connected at least to the first means for detecting (10), to the access zone (8) and to the means for controlling the displacement (P) of the cell and able to act at least on the access zone (8) and/or on the means for controlling the displacement (P) of the cell in order to apply an action to the cell.

11. System for handling according to one of claims 1 to 10 in combination with claim 3, wherein said at least one actuator (14) is a piezoelectric actuator.

12. Method for handling implementing a system for handling biological cells according to one of claims 1 to 11 comprising:
a) the supplying of a solution that contains at least one cell at the inlet end of the main channel,
b) the displacing of the cell in the main channel,
c) the detecting of the presence of the cell at the inlet of the main channel,
d) the determining of at least one property of the cell and optionally the determining of said cell,
e) the decision of applying or not an action to the cell,
f) if an action is to be applied to the cell, the controlling of the means for controlling the displacement of the cell in order to immobilise the cell at the access zone and controlling the access zone in order to apply an action,
g) the controlling of the means for controlling the displacement of the cell in order to bring it to the outlet end,
advantageously at least steps e), f) and g) being controlled by a control unit.

13. Method for handling according to claim 12, with the system for handling comprising second means for detecting between the access zone and the outlet end, wherein a comparison of the signals emitted by the first means for detecting and the signals emitted by the second means for detecting is carried out in order to detect a modification in at least one property of the cell following the action that has been applied to it.

14. Method for handling according to claim 12 or 13, wherein the 'action is a delivery of macromolecules or the action is the collection of one or several substances ejected by the cell, for example secretome.

15. Method for handling according to one of claims 12 to 14, said at least one property of the cell being its deformability, the step d), takes a measurement of said deformability of the cell and makes it possible to deduce if it has a metastatic potential.
